(19) Europäisches Patentamt
European Patent Office
Office européen des brevets

(11) **EP 3 324 823 B1**

(12) **FASCICULE DE BREVET EUROPEEN**

(45) Date de publication et mention
de la délivrance du brevet:
**01.09.2021 Bulletin 2021/35**

(21) Numéro de dépôt: **16747841.1**

(22) Date de dépôt: **15.07.2016**

(51) Int Cl.:
*A61B 3/00* *(2006.01)*  *A61B 3/028* *(2006.01)*
*A61B 3/036* *(2006.01)*

(86) Numéro de dépôt international:
**PCT/FR2016/051827**

(87) Numéro de publication internationale:
**WO 2017/013343 (26.01.2017 Gazette 2017/04)**

(54) **DISPOSITIF DE COMPENSATION VISUELLE, PROCÉDÉ DE COMMANDE D'UN DISPOSITIF DE COMPENSATION VISUELLE ET DISPOSITIF BINOCULAIRE D'OPTOMÉTRIE**

SICHTKOMPENSIERTE VORRICHTUNG, VERFAHREN ZUR STEUERUNG EINER SICHTKOMPENSIERTEN VORRICHTUNG BINOKULARE OPTOMETRIEVORRICHTUNG

VISION-COMPENSATING DEVICE, METHOD FOR CONTROLLING A VISION-COMPENSATING DEVICE AND BINOCULAR OPTOMETRY DEVICE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **17.07.2015 FR 1556795**

(43) Date de publication de la demande:
**30.05.2018 Bulletin 2018/22**

(73) Titulaire: **Essilor International**
**94220 Charenton-le-Pont (FR)**

(72) Inventeur: **BOUTINON, Stéphane**
**94227 Charenton-le-Pont-Cedex (FR)**

(74) Mandataire: **Jacobacci Coralis Harle**
**32, rue de l'Arcade**
**75008 Paris (FR)**

(56) Documents cités:
**WO-A1-2015/107303  WO-A2-2007/026368**
**US-A- 3 927 933  US-A- 4 113 363**
**US-A1- 2004 032 568**

**Description**

DOMAINE TECHNIQUE AUQUEL SE RAPPORTE L'INVENTION

La présente invention concerne les équipements d'optométrie, en particulier destinés à la réfraction subjective.
Elle concerne plus particulièrement un dispositif de compensation visuelle, un procédé de commande d'un dispositif de compensation visuelle et un dispositif binoculaire d'optométrie.

ARRIERE-PLAN TECHNOLOGIQUE

Dans le cadre de la réfraction subjective, on utilise de manière générale un dispositif de compensation visuelle permettant d'observer selon un axe optique d'observation avec une correction optique de puissance variable.
On connaît, par exemple du document US 2004/032 568, un tel dispositif comprenant une lentille ayant, selon l'axe optique, une puissance sphérique variable en fonction d'une première commande et un ensemble optique générant, selon l'axe optique, une correction cylindrique variable en fonction d'au moins une seconde commande appliquée audit ensemble optique.
On propose par exemple dans un tel système d'afficher sur un écran les valeurs de correction optique obtenues par l'application des commandes courantes, ce qui permet au praticien de modifier les commandes pour obtenir d'autres valeurs de correction optique.
Cette solution n'est toutefois pas pratique puisqu'elle oblige le praticien à rechercher par tâtonnement les valeurs de correction visuelle qu'il souhaite tester lors du processus de réfraction subjective.
WO-A-2007/026 368 décrit un système optométrique - ophtalmique multifonctionnel comprenant un ensemble de correction de réfraction tournant une correction de type sphérique, une correction de type cylindrique, une correction de type prismatique ou une combinaison de ceux-ci.

OBJET DE L'INVENTION

Dans ce contexte, la présente invention propose un dispositif de compensation visuelle permettant d'observer selon un axe optique d'observation avec une correction optique de puissance variable, comprenant une lentille ayant, selon l'axe optique, une puissance sphérique variable en fonction d'une première commande, et un ensemble optique générant, selon l'axe optique, une correction cylindrique variable en fonction d'au moins une seconde commande appliquée audit ensemble optique, caractérisé par un module de réception d'au moins une consigne pour ladite correction optique, et par un module de détermination de la première commande et de la seconde commande en fonction de ladite consigne au moyen d'un modèle tenant compte de la distance séparant ladite lentille et ledit ensemble optique.
Grâce à la prise en compte de la distance susmentionnée, c'est-à-dire de l'espacement entre la lentille et l'ensemble optique, on tient compte de phénomènes de couplage générés par cet espacement et on obtient, après application de la première commande et de la seconde commande respectivement à la lentille et au système optique, une correction qui correspond précisément à la consigne (c'est-à-dire à la correction souhaitée par le praticien).
Le module de détermination de la première commande et de la seconde commande peut comprendre en outre un module de détermination d'une première valeur de commande approchée et d'une seconde valeur de commande approchée en fonction de ladite consigne, un module d'évaluation, sur la base dudit modèle, d'au moins une valeur de correction obtenue en appliquant la première valeur de commande approchée à la lentille et la seconde valeur de commande approchée à l'ensemble optique, et un module de détermination d'une première valeur de commande corrigée et d'une seconde valeur de commande corrigée sur la base d'une comparaison entre la consigne et la valeur de correction évaluée.
Le module de détermination de la première commande et de la seconde commande peut alors utiliser la première valeur de commande corrigée et la seconde valeur de commande corrigée respectivement en tant que première commande et seconde commande.
On obtient ainsi en temps réel des valeurs de commandes permettant d'obtenir les valeurs de consigne désirées.
Selon un autre mode de réalisation envisageable, le module de détermination de la première commande et de la seconde commande peut être conçu pour lire la première commande (ainsi qu'éventuellement la seconde commande) dans une table de correspondance construite sur la base dudit modèle.
Dans certains modes de réalisation, l'ensemble optique peut comprendre une seconde lentille et une troisième lentille ; le modèle peut dans ce cas tenir compte également de la distance séparant la seconde lentille et la troisième lentille.
L'invention propose aussi un procédé de commande d'un dispositif de compensation visuelle permettant d'observer selon un axe optique d'observation avec une correction optique de puissance variable et comprenant une lentille et un ensemble optique, caractérisé en ce qu'il comprend les étapes suivantes :

- réception d'au moins une consigne pour ladite correction optique ;
- détermination d'une première commande et d'une seconde commande en fonction de ladite consigne au moyen d'un modèle tenant compte de la distance séparant ladite lentille et ledit ensemble optique ;
- modification de la puissance sphérique de la lentille selon l'axe optique en fonction de la première commande ; et
- modification d'une correction cylindrique générée selon l'axe optique par l'ensemble optique en fonction de la seconde commande.

L'étape de détermination d'une première commande et d'une seconde commande peut comprendre les sous-étapes suivantes :

- détermination d'une première valeur de commande approchée et d'une seconde valeur de commande approchée en fonction de ladite consigne ;
- évaluation, sur la base dudit modèle, d'au moins une valeur de correction obtenue en appliquant la première valeur de commande approchée à la lentille et la seconde valeur de commande approchée à l'ensemble optique ;
- détermination d'une première valeur de commande corrigée et d'une seconde valeur de commande corrigée sur la base d'une comparaison entre la consigne et la valeur de correction évaluée.

Le procédé de commande peut alors éventuellement comprendre les sous-étapes suivantes :

- évaluation, sur la base dudit modèle, d'au moins une nouvelle valeur de correction obtenue en appliquant la première valeur de commande corrigée à la lentille et la seconde valeur de commande corrigée à l'ensemble optique ;
- détermination d'une nouvelle première valeur de commande corrigée et d'une nouvelle seconde valeur de commande corrigée sur la base d'une comparaison entre la consigne et la nouvelle valeur de correction évaluée.

Dans ce cas, les sous-étapes d'évaluation d'au moins une nouvelle valeur de correction et de détermination d'une nouvelle première valeur de commande corrigée et d'une nouvelle seconde valeur de commande corrigée peuvent être réitérées tant que la distance entre la consigne et la nouvelle valeur de correction évaluée est supérieure à un seuil prédéterminé.

Selon la variante mentionnée plus haut, l'étape de détermination d'une première commande et d'une seconde commande peut comprendre une sous-étape de lecture de la première commande (ainsi qu'éventuellement de la seconde commande) dans une table de correspondance construite sur la base dudit modèle.

L'invention propose également un dispositif binoculaire d'optométrie comprenant deux dispositifs optiques, montés par exemple sur un support commun, dans lequel un des deux dispositifs optiques (voire chacun des deux dispositifs optiques) est un dispositif de compensation visuelle comme présenté ci-dessus.

DESCRIPTION DETAILLEE D'UN EXEMPLE DE REALISATION

La description qui va suivre en regard des dessins annexés, donnés à titre d'exemples non limitatifs, fera bien comprendre en quoi consiste l'invention et comment elle peut être réalisée.

Sur les dessins annexés :

- la figure 1 représente schématiquement les éléments optiques utilisés dans un exemple de mise en œuvre de l'invention ;
- la figure 2 représente une vue en coupe d'un exemple de dispositif de compensation visuelle conforme aux enseignements de l'invention ;
- la figure 3 représente une vue écorchée du dispositif de compensation de la figure 2 côté lentilles cylindriques ;
- la figure 4 est une vue écorchée du dispositif de compensation de la figure 2 côté lentille sphérique variable ;
- la figure 5 représente schématiquement un élément de commande du dispositif de compensation visuelle de la figure 2 ;
- la figure 6 représente un exemple de construction possible pour un module de calcul de l'élément de commande de la figure 5.

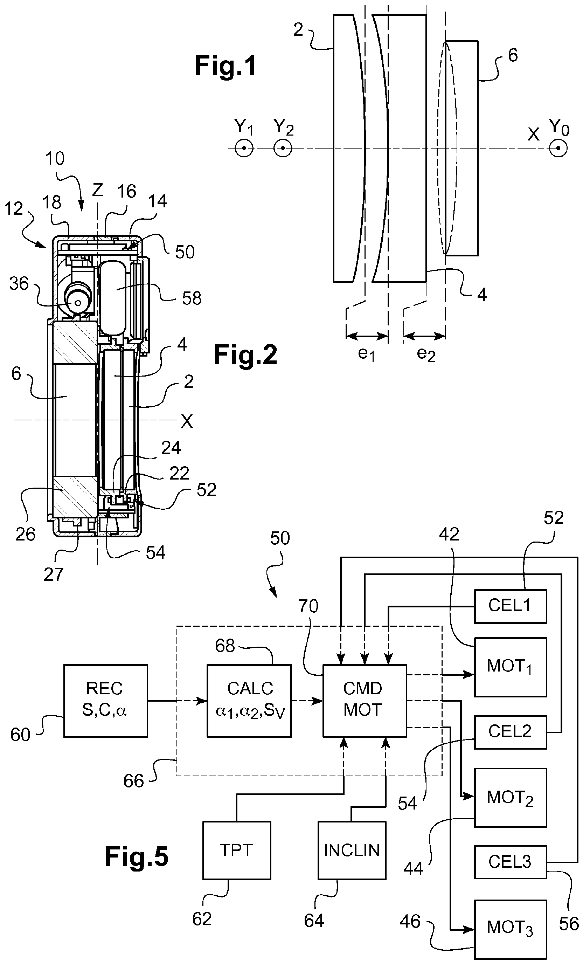

Sur la figure 1 sont schématiquement représentés les éléments optiques principaux d'un exemple de dispositif de compensation visuelle conforme aux enseignements de l'invention.

Ces éléments optiques comprennent une lentille plan-cylindre convexe 2, de puissance cylindrique $C_1$ (ici égale à $C_0$), une lentille plan-cylindre concave 4, de puissance cylindrique $C_2$ (ici négative et égale à $-C_0$), et une lentille 6 de puissance sphérique variable $S_V$.

La valeur absolue (ou module), ici $C_0$, de la puissance cylindrique (ici $-C_0$) de la lentille plan-cylindre concave 4 est donc égale à la valeur absolue (C$_0$) (ou module) de la puissance cylindrique (C$_0$) de la lentille plan-cylindre convexe 2.

Ces trois lentilles 2, 4, 6 sont placées sur le même axe optique X. Précisément, chacune des trois lentilles 2, 4, 6 a une forme extérieure généralement cylindrique, centrée sur l'axe optique X. Dans l'exemple décrit ici, les lentilles 2, 4, 6 ont respectivement les diamètres (mesurant leur encombrement) suivants : 25 mm, 25 mm, 20 mm.

On remarque de ce fait qu'il est préférable d'utiliser ce dispositif de compensation visuelle 10 en positionnant l'œil du patient du côté de la lentille de puissance sphérique variable 6 de sorte que les lentilles de puissance cylindrique 2, 4, de plus grand diamètre, ne viennent pas limiter le champ de vision défini par la lentille de puissance sphérique variable 6, qui est lui-même large du fait de la proximité de l'œil du patient.

Chacune des trois lentilles 2, 4, 6 comporte une première face plane, perpendiculaire à l'axe optique X, et une seconde face, opposée à la première face et optiquement active :

- la face optiquement active de la lentille 2 est de forme cylindrique convexe (l'axe Y$_1$ du cylindre définissant cette face étant perpendiculaire à l'axe optique X) ;
- la face optiquement active de la lentille 4 est de forme cylindrique concave (l'axe Y$_2$ du cylindre définissant cette face étant perpendiculaire à l'axe optique X) ;
- la face optiquement active de la lentille 6 de puissance sphérique variable S$_V$ est déformable et peut ainsi prendre une forme sphérique convexe (comme illustré en pointillés sur la figure 1), une forme plane (comme illustré en trait plein) ou une forme sphérique concave (comme illustré en trait mixte).

La lentille 6 de puissance sphérique variable S$_V$ est par exemple une lentille du type décrit dans le document EP 2 034 338. Une telle lentille comprend une cavité fermée par une membrane déformable transparente et une paroi plane transparente mobile ; la cavité contient un liquide transparent de volume constant qui est plus ou moins contraint par la face mobile, afin de déformer la membrane qui est de ce fait soit une surface concave sphérique, soit une surface plane, soit une surface convexe sphérique. Dans la lentille utilisée, une transformation de mouvement réalisée par un système vis écrou permet d'assurer la transformation de mouvement translation - rotation. Ainsi, une rotation d'une bague montée sur un boîtier 26 entraîne en translation une pièce de la lentille 6, ce qui provoque la déformation susmentionnée de la membrane transparente comme expliqué par exemple dans le document EP 2 034 338 précité. On peut ainsi faire varier continûment la puissance sphérique S$_V$ par action mécanique sur la lentille 6. Dans l'exemple décrit ici, la lentille 6 a une focale variable entre -40 mm et 40 mm, soit une puissance sphérique S$_V$ variable entre -25D et 25D (D étant la dioptrie, unité de mesure de la vergence, inverse de la focale exprimée en mètres).

Par ailleurs, les lentilles plan-cylindre 2, 4 ont respectivement comme déjà indiqué une puissance cylindrique -C$_0$ et C$_0$, ici avec C$_0$ = 5D.

Comme expliqué plus en détail dans la suite, la lentille plan-cylindre concave 4 et la lentille plan-cylindre convexe 2 sont montées en rotation autour de l'axe X (rotation centrée sur l'axe X).

L'axe Y$_1$ du cylindre convexe formé sur la face optiquement active de la lentille plan-cylindre convexe 2 peut ainsi former un angle variable $\alpha_1$ avec un axe de référence Y$_0$ (fixe et perpendiculaire à l'axe optique X).

De même, l'axe Y$_2$ du cylindre concave formé sur la face optiquement active de la lentille plan-cylindre concave 4 peut former un angle variable $\alpha_2$ avec l'axe de référence Y$_0$.

La lentille plan-cylindre convexe 2 et la lentille plan-cylindre concave 4 sont espacées d'une distance e$_1$ selon l'axe optique ; la lentille plan-cylindre concave 4 et la lentille 6 de puissance sphérique variable S$_V$ sont espacées d'une distance e$_2$ selon l'axe optique. Dans le mode de réalisation décrit plus bas en référence à la figure 2, e$_1$ vaut par exemple (environ) 1 mm (de manière générale, e$_1$ peut être compris entre 0,5 mm et 2 mm) et e$_2$ vaut par exemple (environ) 5mm (de manière générale, e$_2$ peut être compris entre 2 mm et 10 mm).

Afin d'expliquer de manière simple le comportement optique du système qui vient d'être décrit, on donne ci-dessous les formules pour la puissance sphérique S, la puissance cylindrique C et l'angle d'astigmatisme $\alpha$ du système formé des trois éléments optiques 2, 4, 6, ces formules étant obtenues par calcul de la vergence sur les différents méridiens dans un modèle où on néglige le phénomène de couplage causé par les espacements e$_1$, e$_2$ entre les différentes lentilles :

$$\tan 2\alpha = \frac{\sin 2\alpha_2 - \sin 2\alpha_1}{\cos 2\alpha_2 - \cos 2\alpha_1} = -\frac{\cos(\alpha_1 + \alpha_2)}{\sin(\alpha_1 + \alpha_2)} \text{ (formule 1)}$$

$$C = C_0\left(\cos 2(\alpha - \alpha_2) - \cos 2(\alpha - \alpha_1)\right) \text{ (formule 2)}$$

$$S = S_V - \frac{C}{2} \text{ (formule 3).}$$

On remarque que le terme (-C/2) dans la formule 3 correspond à puissance sphérique générée par la résultante des 2 lentilles à puissance cylindrique.

En pilotant la position en rotation de la lentille plan-cylindre convexe 2 et la position en rotation de la lentille plan-cylindre concave 4, indépendamment l'une de l'autre, comme décrit ci-après, on peut faire varier indépendamment chacun des angles $\alpha_1$, $\alpha_2$ de 0° à 360° et ainsi obtenir une puissance cylindrique C réglable entre $-2.C_0$ et $2.C_0$ (soit ici entre -10D et 10D), et pour n'importe quel angle d'astigmatisme réglable entre 0° et 360° obtenu par une commande simultanée des deux lentilles. Comme l'indique la formule numéro 3, la résultante de puissance sphérique induite par la résultante de l'orientation des 2 lentilles cylindriques est compensée à l'aide de la lentille sphérique de puissance variable.

Par ailleurs, en faisant varier la puissance sphérique $S_V$ de la lentille sphérique 6, on peut régler la puissance sphérique S du système formé des trois lentilles 2, 4, 6.

Selon une variante envisageable, les lentilles à puissance cylindrique fixe pourraient avoir la même puissance cylindrique $C_0$ (positive ou négative) : il pourrait s'agir de deux lentilles plan-cylindre convexe, éventuellement identiques, ou, en alternative, de deux lentilles plan-cylindre concave, éventuellement identiques.

En effet, dans ce cas, la puissance sphérique S, la puissance cylindrique C et l'angle d'astigmatisme $\alpha$ du système formé de ces deux lentilles et d'une lentille à puissance sphérique variable sont donnés par les formules suivantes :

$$\tan 2\alpha = \frac{\sin 2\alpha_2 + \sin 2\alpha_1}{\cos 2\alpha_2 + \cos 2\alpha_1} \text{ (formule 4)}$$

$$C = C_0 \left( \cos 2(\alpha - \alpha_2) + \cos 2(\alpha - \alpha_1) \right) \text{ (formule 5)}$$

$$S = S_V + C_0 - \frac{C}{2}. \text{ (formule 6)}$$

Le terme $C_0$ - C/2 correspond à la puissance sphérique induite par la combinaison des deux lentilles à puissance cylindrique.

On peut donc également dans ce cas régler la puissance sphérique S, la puissance cylindrique C et l'angle d'astigmatisme $\alpha$, en particulier de sorte que la puissance cylindrique C soit nulle, en entraînant en rotation les lentilles à puissance cylindrique (indépendamment l'une de l'autre) et en faisant varier la puissance sphérique de la lentille à puissance sphérique variable.

Un exemple de dispositif de compensation visuelle 10 qui utilise les éléments optiques qui viennent d'être décrits est représenté en figure 2.

On utilisera parfois dans la description qui suit, afin de clarifier l'explication, des termes, comme "*supérieur*" ou "*inférieur*", qui définissent une orientation dans les figures 2, 3 et 4. On comprend que cette orientation n'est pas nécessairement applicable à l'utilisation qui pourra être faite du dispositif décrit, utilisation dont la seule direction de référence est l'axe optique X.

Le dispositif de compensation visuelle 10 comprend un boîtier 12 formé d'une première partie 14, d'une seconde partie 16 et d'une troisième partie 18, qui s'étendent successivement selon l'axe optique X et sont assemblées deux à deux au niveau de plans perpendiculaires à l'axe optique X.

Une première roue dentée 22 est montée en rotation centrée sur l'axe optique X dans la première partie 14 du boîtier 12 et porte en son centre, dans une ouverture prévue à cet effet, la lentille plan-cylindre convexe 2. La première roue dentée 22 et la lentille plan-cylindre convexe 2 sont coaxiales ; autrement dit, en section dans un plan perpendiculaire à l'axe optique X, la circonférence extérieure de la première roue dentée 22 et la circonférence de la lentille plan-cylindre convexe 2 forment des cercles concentriques centrés sur l'axe optique X.

De même, une seconde roue dentée 24 est montée en rotation centrée sur l'axe optique X dans la seconde partie 16 du boîtier 12 et porte en son centre, dans une ouverture prévue à cet effet, la lentille plan-cylindre concave 4. La seconde roue dentée 24 et la lentille plan-cylindre concave 4 sont coaxiales ; autrement dit, en section dans un plan perpendiculaire à l'axe optique X, la circonférence extérieure de la seconde roue dentée 24 et la circonférence de la lentille plan-cylindre concave 4 forment des cercles concentriques centrés sur l'axe optique X.

Une troisième roue dentée 27 est montée en rotation centrée sur l'axe optique X dans la troisième partie 18 du boîtier 12. La troisième roue dentée 27 est solidaire de la bague pourvue sur la circonférence du boîtier 26 qui porte la lentille 6 de puissance sphérique variable et permettant la commande de la puissance sphérique $S_V$. Le boîtier 26 de la lentille 6 de puissance sphérique variable est monté dans la troisième partie 18 du boîtier 12.

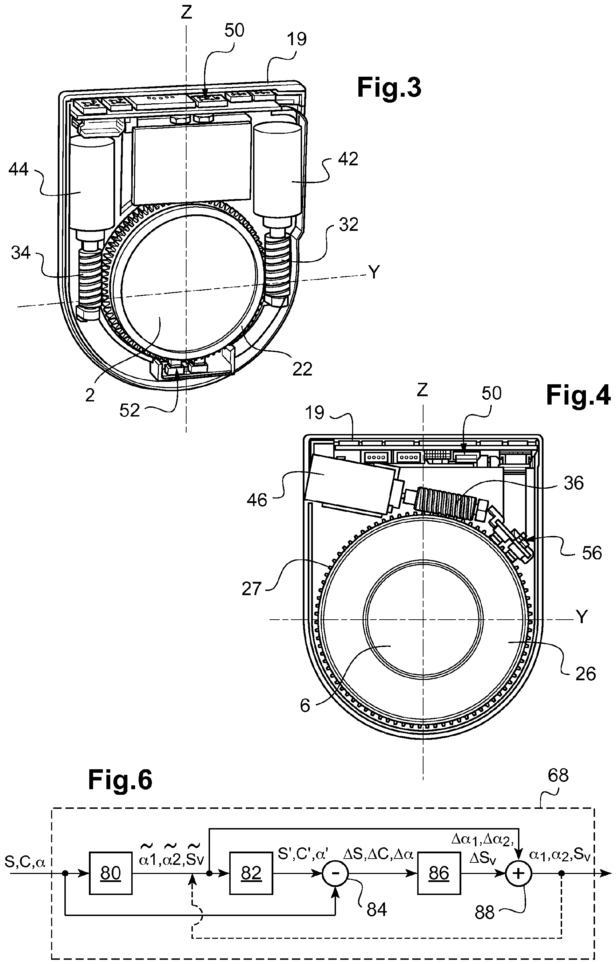

Comme bien visible en figure 3, la première roue dentée 22 est entraînée en rotation (autour de l'axe optique X) au moyen d'un premier moteur 42 dont un axe d'entraînement porte une première vis sans fin 32 qui engrène sur la première roue dentée 22. Le premier moteur 42 est par exemple monté dans la première partie 14 du boîtier 12.

La position courante de la première roue dentée 22 est surveillée par une première cellule optique 52.

De même, la seconde roue dentée 24 est entraînée en rotation autour de l'axe optique X au moyen d'un second moteur 44 dont un axe d'entraînement porte une seconde vis sans fin 34 qui engrène sur la seconde roue dentée 24. Le second moteur 44 est par exemple monté dans la seconde partie 16 du boîtier 12.

La position courante de la seconde roue dentée 24 est surveillée par une seconde cellule optique 54.

Comme représenté sur la figure 4, la troisième roue dentée 27 est quant à elle entraînée en rotation (autour de l'axe optique X) au moyen d'un troisième moteur 46 qui présente un axe d'entraînement sur lequel est monté une troisième vis sans fin 36 qui engrène avec la troisième roue dentée 27. Le troisième moteur 46 est par exemple monté dans la troisième partie 18 du boîtier 12.

La position courante de la troisième roue dentée 27 est surveillée par une troisième cellule optique 56.

Chaque cellule optique 52, 54, 56 est par exemple formée d'un couple d'éléments comprenant au moins un capteur optique ; l'autre élément du couple est par exemple un émetteur optique (ou, en variante, un élément réfléchissant, auquel cas un émetteur optique est associé au capteur optique).

Les premier, second et troisième moteurs 42, 44, 46 sont par exemple des moteurs pas à pas, d'une résolution de 20 pas/tour, pilotés ici en 8$^{ème}$ de pas (ci-après micro-pas). En variante, ces moteurs pourraient être pilotés en 16$^{ème}$ de pas. En variante, il pourrait s'agir de moteurs à courant continu avec des codeurs.

Le volume interne du boîtier 12 (comme d'ailleurs le volume interne de chacune des première, seconde et troisième parties 14, 16, 18 de la même manière) peut être subdivisé en un espace de réception des moteurs 42, 44, 46 (région supérieure du boîtier 12 sur les figures 2, 3 et 4) et un espace de réception des éléments optiques 2, 4, 6 (région inférieure du boîtier 12 sur les figures 2, 3 et 4).

L'espace de réception des moteurs 42, 44, 46 a une forme essentiellement parallélépipédique, ouverte (vers le bas sur les figures) en direction de l'espace de réception des éléments optiques 2, 4, 6 et fermé à l'opposé (vers le haut sur les figures) par une face supérieure 19 du boîtier 12 (la face supérieure 19 du boîtier 12 étant formée par l'assemblage de faces supérieures respectives des première, seconde et troisième parties 14, 16, 18 du boîtier 12).

La disposition des moteurs 42 44 et 46 est telle qu'elle permet de bénéficier d'une géométrie circulaire sur 180° centrée sur l'axe optique au plus proche du rayon utile des lentilles.

L'espace de réception des éléments optiques 2, 4, 6 présente, à l'opposé de l'espace de réception des moteurs, une forme cylindrique (délimitée par les parois du boîtier 12) qui épouse celle de la troisième roue dentée 27 sur la moitié de la circonférence de celle-ci.

Autrement dit, le boîtier 12 (et par conséquent chacune des première, seconde et troisième parties 14, 16, 18 du boîtier 12) a, au niveau de l'espace de réception des éléments optiques 2, 4, 6, une forme cylindrique de diamètre (perpendiculairement à l'axe optique X) du même ordre que, et légèrement supérieur à, celui de la troisième roue dentée 27.

Les diamètres respectifs des roues dentées 22, 24, 27 sont adaptés de manière à favoriser la conservation du champ en dépit de l'épaisseur du système optique.

Le premier moteur 42 et la première vis sans fin 32 s'étendent dans le boîtier 12 selon une direction Z perpendiculaire à la face supérieure du boîtier 12 (et donc notamment perpendiculaire à l'axe optique X) de telle sorte que le premier moteur 42 est logé dans l'espace de réception des moteurs tandis que la première vis sans fin 32 s'étend dans l'espace de réception des éléments optiques.

Le second moteur 44 et la seconde vis sans fin 34 s'étendent quant à eux dans le boîtier 12 selon la même direction, mais à l'opposé du premier moteur 42 et de la première vis sans fin 34 par rapport aux lentilles de puissance cylindrique 2, 4. Le second moteur 44 est logé dans l'espace de réception des moteurs tandis que la seconde vis sans fin 34 s'étend dans l'espace de réception des éléments optiques.

On remarque qu'ainsi la première vis sans fin 32 et la seconde vis sans fin 34 sont situées de part et d'autre de l'ensemble formé par la première roue dentée 22 et la seconde roue dentée 24, et que l'encombrement latéral (selon un axe Y perpendiculaire aux axes X et Z précités) de ces différentes pièces (première vis sans fin 32, seconde vis sans fin 34, première ou seconde roue dentée 22, 24) est inférieur au diamètre de la troisième roue dentée 27 de sorte que les première et seconde vis sans fin 32, 34 contiennent dans l'espace de réception des éléments optiques sans nécessiter d'excroissance pour les accueillir.

Par ailleurs, les premier et second moteurs 42, 44 ont chacun un encombrement selon l'axe optique X supérieur à celui de chacune des première et seconde roues dentées 22, 24, et même supérieur à celui de chacune des première et seconde parties 14, 16 de boîtier 12. Toutefois, du fait que ces premier et second moteurs 42, 44 sont placés comme il vient d'être indiqué de chaque côté du boîtier 12 (par rapport à l'axe Z), ils peuvent chacun occuper un espace qui s'étend selon l'axe optique X au droit de la première partie 14 et de la seconde partie 16 du boîtier 12.

Par exemple, chacun des premier et second moteurs 42, 44 a un encombrement latéral (diamètre externe du moteur) compris entre 6 et 12, par exemple 10 mm, tandis que les première et seconde roues dentées 22, 24 ont chacune une épaisseur (encombrement selon l'axe X) compris entre 1 et 4, par exemple 2,5 mm.

Le troisième moteur 46 et la troisième vis sans fin 36 sont en revanche situés dans l'espace de réception des moteurs, dans la région qui s'étend selon l'axe X au droit de la troisième partie 18 du boîtier 12. Ainsi, la troisième vis sans fin 36 engrène la troisième roue dentée 27 dans une partie supérieure de celle-ci, ce qui permet au boîtier 12 d'épouser la forme du boîtier 12 dans la partie inférieure de la troisième roue dentée 27, comme déjà indiqué.

Dans l'exemple décrit, comme visible en figure 4, l'axe du troisième moteur 46 et de la troisième vis sans fin 36 est légèrement incliné par rapport à la face supérieure du boîtier 12 (précisément par rapport à l'axe Y précité).

On prévoit par exemple que l'épaisseur de la troisième roue dentée 27 est comprise entre 0,3 mm et 2 mm.

Cette disposition des différents éléments permet d'obtenir un boîtier relativement fin, ayant typiquement une épaisseur comprise entre 15 et 20 mm.

Le boîtier 12 comprend également, par exemple dans la région supérieure de l'espace de réception des moteurs, un élément de commande 50, formé ici de plusieurs circuits intégrés portés par un circuit imprimé commun.

Par ailleurs un dispositif de stockage d'énergie électrique de type batterie 58 (ou, en variante, une super capacité) est prévu pour rendre l'appareil autonome. On prévoit par exemple également des éléments de recharge sans contact du dispositif de stockage d'énergie 58. La batterie 58 permet notamment l'alimentation électrique des moteurs 42, 44, 46 et de l'élément de commande 50.

Les éléments principaux d'un tel élément de commande 50, ainsi que leur connexion aux moteurs 42, 44, 46 précités et aux cellules optiques 52, 54, 56 précitées, sont représentés schématiquement en figure 5.

L'élément de commande 50 comprend un module de réception 60 conçu pour recevoir, ici à travers une liaison sans fil, les informations de consigne, c'est-à-dire des informations indicatives des valeurs souhaitées par l'utilisateur pour la puissance sphérique S, la puissance cylindrique C et l'angle d'astigmatisme $\alpha$ qui définissent la compensation générée par le système optique formé des éléments optiques 2, 4, 6.

Le module de réception 60 est par exemple un module de réception infrarouge qui reçoit ces informations de consigne d'une télécommande à émission infrarouge manipulée par l'utilisateur. En variante, on pourrait prévoir que ces informations de consigne soient reçues d'un ordinateur personnel via une liaison sans fil, par exemple un réseau local sans fil ; l'utilisateur pourrait dans ce cas choisir des valeurs de puissance sphérique S, de puissance cylindrique C et d'angle d'astigmatisme $\alpha$ pour le dispositif de compensation visuelle par sélection interactive sur l'ordinateur.

Le module de réception 60 transmet les informations de consigne S, C, $\alpha$ reçues à un calculateur 66 (constitué par exemple d'un processeur exécutant un programme d'ordinateur de manière à mettre en œuvre les fonctions du calculateur décrites ci-après), précisément à un module de conversion 68 mis en œuvre par ce calculateur 66.

Le module de conversion 68 détermine les valeurs des angles $\alpha_1$, $\alpha_2$ et la valeur de puissance sphérique $S_V$ nécessaires afin d'obtenir les valeurs de consigne S, C, $\alpha$ reçues en entrée, conformément à ce qui est décrit plus loin en référence à la figure 6.

Le calculateur 66 met également en œuvre un module de commande 70 qui reçoit en entrée les valeurs d'angle $\alpha_1$, $\alpha_2$ et de puissance sphérique $S_V$ calculées par le module de conversion 68 et émet des signaux de commande à destination des moteurs 42, 44, 46 afin de commander chacun des moteurs 42, 44, 46 indépendamment des autres de manière à obtenir des positionnements respectifs des roues dentées 22, 24, 27 qui permettent d'obtenir les valeurs souhaitées :

- le module de commande 70 commande le premier moteur 42 de manière à faire tourner la première roue dentée 22 autour de l'axe optique X jusqu'à la position où l'axe $Y_1$ de la surface cylindrique optiquement active de la lentille plan-cylindre convexe 2 (portée par la première roue dentée 22) forme un angle $\alpha_1$ avec la direction de référence $Y_0$ ;
- le module de commande 70 commande le second moteur 44 de manière à faire tourner la seconde roue dentée 24 autour de l'axe optique X jusqu'à la position où l'axe $Y_2$ de la surface cylindrique optiquement active de la lentille plan-cylindre concave 4 (portée par la seconde roue dentée 24) forme un angle $\alpha_2$ avec la direction de référence $Y_0$ ;
- le module de commande 70 commande le troisième moteur 46 de manière à faire tourner la troisième roue dentée 27 autour de l'axe optique X jusqu'à la position où la bague de commande de la puissance sphérique variable commande la puissance sphérique $S_V$ calculée par le module de conversion 68.

La position de chaque roue dentée 22, 24, 27 est connue à chaque instant respectivement grâce aux cellules optiques 52, 54, 56 qui mesurent chacune, sur la roue dentée à laquelle chacune est associée, le nombre de dents ayant traversé la cellule optique par rapport à un point de référence sur la circonférence de la roue concernée (par exemple dépourvu de dent).

Dans l'exemple décrit ici, l'ensemble premier moteur 42-première vis sans fin 32-première roue dentée 22, comme l'ensemble second moteur 44-seconde vis sans fin 34-seconde roue dentée 24, génère une démultiplication telle qu'un tour de roue dentée 22, 24 correspond à 15040 micro-pas du moteur associé 42, 44. La résolution (angle de rotation des roues dentées 22, 24 pour un micro-pas) est donc de 0,024° pour les angles $\alpha_1$ et $\alpha_2$.

L'ensemble troisième moteur 46-troisième vis sans fin 36-troisième roue dentée 46 génère quant à lui une démultiplication de 16640 micro-pas par tour. La bague de commande de la puissance sphérique variable est réglable sur une plage angulaire de 120° (ce qui correspond donc à 5547 micro-pas) afin d'obtenir la variation de puissance sphérique de -25D à 25D (soit une plage de variation de 50D). La résolution (variation de puissance sphérique $S_V$ pour un micro-pas) est donc de 0,009D.

Selon un mode de réalisation envisageable, on peut prévoir que l'élément de commande 50 prenne en compte la distance entre la face d'entrée de la lentille sphérique 6 et le sommet de la cornée d'un œil observant à travers le dispositif de compensation visuelle, afin de corriger éventuellement les consignes en puissance du dispositif de compensation visuelle concerné. Cette distance (parfois dénommée DVO, pour "*distance verre-œil*") peut être obtenue par des moyens connus pour ce faire.

En prenant l'exemple d'une puissance sphérique S de focale équivalente F, une erreur de positionnement $\varepsilon$ revient à avoir une correction de focale F', équivalente à une puissance sphérique S', avec :

$$F' = F - \varepsilon \quad \text{et} \quad S' = S \left( \cfrac{1}{1 - \cfrac{\varepsilon}{F}} \right),$$

ce qui donne en première approximation $S' = S \cdot (1 + \varepsilon \cdot S)$.

L'élément de commande 50 détermine donc, selon ce mode de réalisation, les valeurs des angles $\alpha_1$, $\alpha_2$ et la valeur de puissance sphérique $S_V$ (et les signaux de commandes à appliquer respectivement aux moteurs comme indiqué ci-dessus) non seulement en fonction des valeurs de consigne S, C, $\alpha$ reçues en entrée mais également en fonction de la distance œil - dispositif (ici cornée - face d'entrée de la lentille 6). On remarque que la distance verre-œil est ici prise en compte par l'élément de commande 50, qui reçoit les consignes brutes (c'est-à-dire sans prise en compte de la distance verre-œil).

Par ailleurs, on peut prévoir que, lors du passage de valeurs de consigne initiales $\alpha_1$, $\alpha_2$, $S_V$ à de nouvelles valeurs de consigne $\alpha'_1$, $\alpha'_2$, $S'_V$, chacun des premier, second et troisième moteurs 42, 44, 46 soient actionnés pendant une même durée T (en secondes), qui peut dépendre éventuellement de l'amplitude de l'un des changements de consigne (par exemple de la variation, en valeur absolue, de puissance sphérique $| S'_V - S_V |$, où $| x |$ est la valeur absolue de x).

Pour ce faire, le calculateur 66 détermine par exemple le nombre $p_1$ de micro-pas du moteur 42 permettant le passage de l'angle $\alpha_1$ à l'angle $\alpha'_1$, le nombre $p_2$ de micro-pas du moteur 44 permettant le passage de l'angle $\alpha_2$ à l'angle $\alpha'_2$ et le nombre $p_3$ de micro-pas du moteur 46 permettant le passage de la puissance sphérique $S_V$ à la puissance sphérique $S'_V$. Le calculateur 66 commande alors la rotation du moteur 42 à une vitesse de $p_1/T$ micro-pas par seconde, la rotation du moteur 44 à une vitesse de $p_2/T$ micro-pas par seconde et la rotation du moteur 46 à une vitesse de $p_3/T$ micro-pas par seconde.

L'élément de commande 50 comprend également un capteur de température 62, qui délivre une information de température ambiante mesurée, et un inclinomètre 64, par exemple réalisé sous forme d'un accéléromètre et qui délivre une information d'orientation du dispositif de compensation visuelle 10, par exemple par rapport à la verticale.

Le calculateur 66 reçoit l'information de température en provenance du capteur de température 62 et l'information d'orientation en provenance de l'inclinomètre 64 et utilise ces informations dans le cadre de la détermination des commandes à envoyer aux moteurs 42, 44, 46.

Dans l'exemple décrit, le module de commande 70 utilise l'information de température afin de compenser les variations de puissance sphérique de la lentille 6 dues à la température (qui sont de l'ordre de 0,06D/°C dans l'exemple décrit) et l'information d'orientation afin de compenser les perturbations éventuelles du système d'entraînement (moteurs, vis sans fin, roues dentées) dues à des changements d'orientation du dispositif de compensation visuelle 10.

On décrit à présent en référence à la figure 6 un exemple de construction possible du module de conversion 68.

Comme déjà indiqué, ce module de conversion 68 est conçu pour déterminer les valeurs des angles $\alpha_1$, $\alpha_2$ et de la valeur de puissance sphérique $S_V$ nécessaires afin d'obtenir les valeurs de consigne S, C, $\alpha$ reçues en entrée, ici en utilisant un modèle tenant compte des distances $e_1$, $e_2$ séparant les différentes lentilles.

Comme déjà indiqué pour le calculateur 66, le module de conversion 68 est représenté en figure 6 sous forme de blocs fonctionnels, mais pourrait en pratique être mis en œuvre du fait de l'exécution, par un processeur (par exemple un microprocesseur), d'instructions de programme d'ordinateur.

Le module de conversion 68 comprend un premier bloc 80 qui reçoit en entrée les valeurs de consigne S, C, $\alpha$ et détermine sur cette base des valeurs approchées $\tilde{\alpha}_1$, $\tilde{\alpha}_2$, $\tilde{S}_V$ pour les angles $\alpha_1$, $\alpha_2$ et la puissance sphérique $S_V$, par exemple comme suit :

$$\begin{cases} \tilde{\alpha}_1 = \alpha - \frac{1}{2}\arcsin\left(\frac{C}{2C_0}\right) + \frac{\pi}{4} \\ \\ \tilde{\alpha}_2 = \alpha + \frac{1}{2}\arcsin\left(\frac{C}{2C_0}\right) + \frac{\pi}{4} \end{cases}$$

$$\tilde{S}_V = S + \frac{C}{2}$$

On remarque que ces formules sont basées sur celles données plus haut et ne tiennent pas compte des espacements $e_1$, $e_2$ séparant les différentes lentilles (d'où la désignation des résultats obtenus comme "*valeurs approchées*").

Les valeurs approchées $\tilde{\alpha}_1$, $\tilde{\alpha}_2$, $\tilde{S}_V$ sont transmises à un second bloc 82 ainsi qu'à un bloc additionneur 88.

Le second bloc 82 reçoit en entrée les valeurs approchées et estime les valeurs de puissance sphérique S', de puissance cylindrique C' et d'angle d'astigmatisme $\alpha$' obtenues (pour le système optique formé des deux lentilles cylindriques 2, 4 et de la lentille 6 de puissance sphérique variable) si les valeurs approchées $\tilde{\alpha}_1$, $\tilde{\alpha}_2$, $\tilde{S}_V$ reçues étaient utilisées dans le dispositif. Cette estimation est basée sur un modèle tenant compte des distances $e_1$, $e_2$ séparant les différentes lentilles.

Ici par exemple, en utilisant les formules de Gullstrand, la puissance optique pour chaque méridien (repéré par un angle $\phi$) vaut (pour le système optique formé des deux lentilles cylindriques 2, 4 et de la lentille 6 de puissance sphérique variable) :

$$P(\phi) = S_V + A_1(S_V).P_1(\phi) + A_2(S_V).P_2(\phi) + A_3(S_V).P_1(\phi).P_2(\phi)$$

avec

$$P_1(\phi) = C_1 \sin^2\left(\tilde{\alpha}_1 - \phi\right)$$

$$P_2(\phi) = C_2 \sin^2\left(\tilde{\alpha}_2 - \phi\right)$$

$$A_1(S_V) = 1 + \left(e_1 - e_2 - K\right)\cdot S_V$$

$$A_2(S_V) = 1 - (e_2 + K)\cdot S_V$$

$$A_3(S_V) = -e_1 \cdot \left(1 - \left(K(S_V) + e_2\right)\cdot S_V\right)$$

$$K = w_0 - h \cdot \left(1 - \frac{1}{n_{LV}}\right),$$ où $w_0$ est la flèche de la lentille 6, h l'épaisseur de la lentille 6 et $n_{LV}$ l'indice du liquide remplissant la lentille 6, K la distance entre la position au repos de la membrane et le plan principal objet de la lentille variable.

Les paramètres A1, A2 et A3 sont donc des fonctions variables de $S_V$, tandis que les autres paramètres sont des constantes du système (qui peuvent être étalonnées).

Par définition de la puissance sphérique, de la puissance cylindrique et de l'angle d'astigmatisme du système optique, cette puissance optique P pour chaque méridien s'écrit également : $P(\phi) = S'+C'\sin^2 (\alpha'-\phi)$.

On peut ainsi par exemple obtenir C' et α' en calculant la dérivée $dP/d\phi$ de la fonction $P(\phi)$ et en prenant 2 valeurs particulières (par exemple $\phi=0$ et $\phi=\pi/4$), ce qui permet d'obtenir $\tan 2\alpha'$ et $C'^2$.

La partie constante de $P(\phi)$ donne par ailleurs accès à S' selon l'équation ci-dessus.

Les valeurs de puissance sphérique S', de puissance cylindrique C' et d'angle d'astigmatisme α' générées en sortie du second bloc 82 sont transmises à un bloc soustracteur 84, qui calcule la différence entre chacune de ces valeurs et la valeur de consigne S, C, α correspondante. Le bloc soustracteur 84 émet ainsi en sortie les valeurs suivantes (qui représentent, pour chaque paramètre, l'erreur due à l'utilisation des valeurs approchées) :

$$\Delta S = S - S' \ ; \ \Delta C = C - C' \ ; \ \Delta \alpha = \alpha - \alpha'.$$

Les valeurs d'erreur $\Delta S$, $\Delta C$, $\Delta \alpha$ émises en sortie du bloc soustracteur 84 sont appliquées en entrée d'un troisième bloc 86 conçu pour déterminer les variations respectives $\Delta \alpha_1$, $\Delta \alpha_2$, $\Delta S_V$ des commandes $\alpha_1$, $\alpha_2$, $S_V$ associées à ces valeurs d'erreur $\Delta S$, $\Delta C$, $\Delta \alpha$ (par exemple par linéarisation de l'égalité :

$$S'+C'\sin^2\left(\alpha'-\phi\right) = S_V + A_1(S_V).P_1(\phi) + A_2(S_V).P_2(\phi) + A_3(S_V).P_1(\phi).P_2(\phi)$$

autour des valeurs S', C', α' et $\tilde{\alpha}_1$, $\tilde{\alpha}_2$, $\tilde{S}_V$). Les valeurs de $\Delta S$ sont par exemple obtenues pour $\tilde{\alpha}_1$, $\tilde{\alpha}_2$ et $\tilde{S}_V$ selon respectivement la dérivée de $dS'/d(\tilde{\alpha}_1)$, $dS'/d(\tilde{\alpha}_2)$, et $dS'/d(\tilde{S}_V)$. On procède de manière identique pour $\Delta C$ et $\Delta \alpha$. Puis on résout classiquement le système d'équations obtenu selon des valeurs particulières.

Les variations de commande $\Delta \alpha_1$, $\Delta \alpha_2$, $\Delta S_V$ sont alors appliquées en entrée du bloc additionneur 88 qui reçoit également en entrée, comme déjà indiqué, les valeurs approchées $\tilde{\alpha}_1$, $\tilde{\alpha}_2$ $\tilde{S}_V$ générées par le premier bloc 80.

Ce bloc additionneur 88 génère donc en sortie les valeurs de commandes suivantes :

$$\alpha_1 = \tilde{\alpha}_1 + \Delta \alpha_1 \ ;$$

$$\alpha_2 = \tilde{\alpha}_2 + \Delta \alpha_2 \ ;$$

$$\Delta S_V = \tilde{S}_V + \Delta S_V.$$

Grâce aux calculs effectués ci-dessus, ces valeurs de commande $\alpha_1$, $\alpha_2$, $S_V$ permettent d'obtenir les valeurs de consigne S, C, α, en tenant compte des phénomènes de couplage liés à l'espacement des lentilles, avec une erreur minime liée à l'approximation faite lors de la linéarisation utilisée au sein du troisième bloc 86.

Selon une variante envisageable, comme représenté en pointillés en figure 6, il est possible d'appliquer une ou plusieurs nouvelle(s) itération(s) du processus décrit ci-dessus afin de faire converger chacune des valeurs d'erreur $\Delta S$, $\Delta C$, $\Delta \alpha$ vers 0 (le processus itératif s'arrêtant par exemple lorsque chacune des valeurs d'erreur est inférieure à un seuil prédéterminé). Pour ces itérations ultérieures, les valeurs de commande générées en sortie $\alpha_1$, $\alpha_2$, $S_V$ à l'itération précédente sont utilisées en tant que valeurs approchées $\tilde{\alpha}_1$, $\tilde{\alpha}_2$, $\tilde{S}_V$ à l'itération courante.

On comprend que le processus qui vient d'être décrit permet la détermination en temps réel des valeurs de commande $\alpha_1$, $\alpha_2$, $S_V$ en fonction des valeurs de consigne S, C, α au moyen d'un modèle tenant compte des distances $e_1$, $e_2$ séparant les différentes lentilles 2, 4, 6.

Selon un autre mode de réalisation envisageable, le module de conversion 68 pourrait mémoriser (au sein d'une table de correspondance ou LUT pour "*Look-Up Table*") un grand nombre de triplets ($\alpha_1$, $\alpha_2$, $S_V$) de valeurs de commande et, pour chaque triplet ($\alpha_1$, $\alpha_2$, $S_V$), le triplet de valeurs (S, C, α) obtenues par utilisation des valeurs de commande $\alpha_1$, $\alpha_2$, $S_V$ concernées.

Les triplets de valeurs (S, C, α) associées à un triplet de valeurs de commande ($\alpha_1$, $\alpha_2$, $S_V$) sont calculés au préalable en utilisant un modèle tenant compte des distances séparant les lentilles 2, 4, 6 (par exemple au moyen des équations données ci-dessus) et mémorisés comme déjà indiqué dans le module de conversion 68.

En pratique, on mémorise par exemple des triplets associés à des valeurs possibles pour S et C régulièrement réparties sur les plages de valeur envisageables. On utilise par exemple 160 valeurs de S sur la plage [-20D, 20D] (ce qui correspond à un pas de 0,25D) et 32 valeurs de C sur la plage [0,8D] (ce qui correspond également à un pas de 0,25D) et on traite le paramètre α par simple rotation, ce qui permet de ne mémoriser que 5120 triplets de valeurs de

commande ($\alpha_1$, $\alpha_2$, $S_V$) associés chacun à une paire (S, C).

Lors du fonctionnement, le module de conversion 68 sélectionne, parmi les triplets (S, C, $\alpha$) mémorisés, le triplet dont les valeurs s'approchent le plus des valeurs de consigne S, C, $\alpha$ reçues en entrée ; le module de conversion 68 lit alors le triplet de valeurs de commandes ($\alpha_1$, $\alpha_2$, $S_V$) associé (dans la table de correspondance) au triplet sélectionné et émet les valeurs lues en sortie.

Dans l'exemple pratique qui vient d'être mentionné les triplets ($\alpha_1$, $\alpha_2$, $S_V$) sont mémorisés en association chacun avec une paire (S, C), le module de conversion 68 lit les valeurs ($\alpha_1$, $\alpha_2$, $S_V$) associés à la paire dont les valeurs s'approchent le plus des valeurs de consigne S, C et effectue une correction en rotation pour tenir compte de l'angle $\alpha$.

Selon une variante envisageable, il est possible de tenir compte en outre de la température (afin de compenser, comme indiqué plus haut, les variations de puissance sphérique de la lentille 6 dues à la température). Le module de conversion 68 mémorise par exemple dans ce cas plusieurs tables de correspondance associées chacune à une température donnée. Lors de l'utilisation, le module de conversion 68 sélectionne la table de correspondance associée à l'information de température délivrée par le capteur de température 62 et effectue le traitement décrit ci-dessus en utilisant la table de correspondance sélectionnée.

Selon un autre mode de réalisation envisageable, le module de conversion 68 pourrait déterminer les valeurs des angles $\alpha_1$, $\alpha_2$ et la valeur de puissance sphérique $S_V$ nécessaires afin d'obtenir les valeurs de consigne S, C, $\alpha$ reçues en entrée au moyen d'un système de simulation à lancer de rayon (ou "*ray tracing*" selon l'appellation anglo-saxonne), le lancer de rayon étant effectué dans un environnement où les lentilles 2, 4, 6 sont modélisées à leurs positions respectives et qui tient donc compte des distances séparant ces lentilles 2, 4, 6.

Le dispositif de compensation visuelle 10 peut être utilisé pour réaliser la fonction des cylindres croisés par retournement, également appelés cylindres de Jackson.

Selon un premier exemple, cette fonction peut être utilisée pour vérifier (voire trouver) un angle $\alpha_0$ de correction cylindrique requise (paramètre parfois dénommé "*axe du cylindre*"). On considère ici qu'une valeur de puissance de correction sphérique $S_0$ et une valeur de puissance de correction cylindrique $C_0$ ont été également déterminées au préalable.

La fonction des cylindres croisés par retournement est alors par exemple réalisée en appliquant en alternance rapide deux ensembles de consignes, soit un premier ensemble de consignes correspondant à un ajout de puissance cylindrique $C_{var}$ (par exemple 0,5D) à 45° de l'axe défini par l'angle $\alpha_0$ :

- une consigne d'angle d'astigmatisme $\alpha_1 = \alpha_0 + 0{,}5.\text{atan}(C_{var}/C_0)$ ;
- une consigne de puissance cylindrique $C_1 = \text{Racine}(C_0^2 + C_{var}^2)$, où Racine est la fonction racine carrée ;
- une consigne de puissance sphérique $S_1 = S_0 + C_0/2 - C_1/2$,

et un second ensemble de consignes correspondant à un ajoût de puissance cylindrique $-C_{var}$ à 45° de l'axe défini par l'angle $\alpha_0$ :

- une consigne d'angle d'astigmatisme $\alpha_2 = \alpha_0 - 0{,}5.\text{atan}(C_{var}/C_0)$ ;
- une consigne de puissance cylindrique $C_2 = \text{Racine}(C_0^2 + C_{var}^2)$ ;
- une consigne de puissance sphérique $S_2 = S_0 + C_0/2 - C_2/2$.

Selon un second exemple, cette fonction peut être utilisée pour vérifier (voire trouver) la valeur de la puissance de correction cylindrique $C_0$ requise. On considère ici qu'une valeur de puissance de correction sphérique $S_0$ et une valeur d'angle d'astigmatisme $\alpha_0$ ont été également déterminées au préalable.

La fonction des cylindres croisés par retournement est alors par exemple réalisée en appliquant en alternance rapide deux ensembles de consignes, soit un premier ensemble de consignes correspondant à un ajoût de puissance cylindrique $C_{var}$ (par exemple 0,5D) dans l'axe défini par l'angle $\alpha_0$ :

- une consigne d'angle d'astigmatisme $\alpha_1 = \alpha_0$ ;
- une consigne de puissance cylindrique $C_1 = C_0 + C_{var}$ ;
- une consigne de puissance sphérique $S_1 = S_0 - C_{var}/2$,

et un second ensemble de consignes correspondant à un ajoût de puissance cylindrique $-C_{var}$ dans l'axe défini par l'angle $\alpha_0$ :

- une consigne d'angle d'astigmatisme $\alpha_2 = \alpha_0$ ;
- une consigne de puissance cylindrique $C_2 = C_0 - C_{var}$ ;
- une consigne de puissance sphérique $S_2 = S_0 + C_{var}/2$.

**Revendications**

1. Dispositif de compensation visuelle permettant d'observer selon un axe optique (X) d'observation avec une correction optique de puissance variable, comprenant :

   - une lentille (6) ayant, selon l'axe optique, une puissance sphérique variable en fonction d'une première commande ($S_V$) ; et
   - un ensemble optique (2, 4) générant, selon l'axe optique, une correction cylindrique variable en fonction d'au moins une seconde commande ($\alpha_1$, $\alpha_2$) appliquée audit ensemble optique (2,4),
   - un module de réception (60) d'au moins une consigne (S, C, $\alpha$) pour ladite correction optique ;
   - un module de détermination (68) de la première commande ($S_V$) et de la seconde commande ($\alpha_1$, $\alpha_2$) en fonction de ladite consigne (S, C, $\alpha$) **caractérisé en ce que** le module de détermination est configuré pour déterminer la première et la deuxième commande au moyen d'un modèle tenant compte de la distance ($e_2$) séparant ladite lentille (6) et ledit ensemble optique (2, 4).

2. Dispositif de compensation visuelle selon la revendication 1, dans lequel le module de détermination (68) de la première commande ($S_V$) et de la seconde commande ($\alpha_1$, $\alpha_2$) comprend :

   - un module (80) de détermination d'une première valeur de commande approchée ($\tilde{S}_V$) et d'une seconde valeur de commande approchée ($\tilde{\alpha}_1$, $\tilde{\alpha}_2$) en fonction de ladite consigne (S, C, $\alpha$) ;
   - un module (82) d'évaluation, sur la base dudit modèle, d'au moins une valeur de correction (S', C', $\alpha$') obtenue en appliquant la première valeur de commande approchée ($\tilde{S}_V$) à la lentille (6) et la seconde valeur de commande approchée ($\tilde{\alpha}_1$, $\tilde{\alpha}_2$) à l'ensemble optique (2, 4) ;
   - un module (84, 86, 88) de détermination d'une première valeur de commande corrigée ($\alpha_1$, $\alpha_2$) et d'une seconde valeur de commande corrigée ($S_V$) sur la base d'une comparaison ($\Delta$S, $\Delta$C, $\Delta\alpha$) entre la consigne (S, C, $\alpha$) et la valeur de correction évaluée (S', C', $\alpha$').

3. Dispositif de compensation visuelle selon la revendication 2, dans lequel le module de détermination (68) de la première commande et de la seconde commande est conçu pour utiliser la première valeur de commande corrigée ($\alpha_1$, $\alpha_2$) et la seconde valeur de commande corrigée ($S_V$) respectivement en tant que première commande et seconde commande.

4. Dispositif de compensation visuelle selon la revendication 1, dans lequel le module de détermination (68) de la première commande et de la seconde commande est conçu pour lire la première commande dans une table de correspondance construite sur la base dudit modèle.

5. Dispositif de compensation visuelle selon l'une des revendications 1 à 4, dans lequel l'ensemble optique comprend une seconde lentille (2) et une troisième lentille (4) et dans lequel le modèle tient compte de la distance ($e_1$) séparant la seconde lentille (2) et la troisième lentille (4).

6. Procédé de commande d'un dispositif de compensation visuelle permettant d'observer selon un axe optique (X) d'observation avec une correction optique de puissance variable et comprenant une lentille (6) et un ensemble optique (2, 4), comprenant les étapes suivantes :

   - réception d'au moins une consigne (S, C, $\alpha$) pour ladite correction optique ;
   - détermination d'une première commande ($S_V$) et d'une seconde commande ($\alpha_1$, $\alpha_2$) en fonction de ladite consigne (S, C, $\alpha$) au moyen d'un modèle tenant compte de la distance ($e_2$) séparant ladite lentille (6) et ledit ensemble optique (2, 4) ;
   - modification de la puissance sphérique de la lentille (6) selon l'axe optique en fonction de la première commande ($S_V$) ; et
   - modification d'une correction cylindrique générée selon l'axe optique par l'ensemble optique (2, 4) en fonction de la seconde commande ($\alpha_1$, $\alpha_2$).

7. Procédé de commande selon la revendication 6, dans lequel l'étape de détermination d'une première commande ($S_V$) et d'une seconde commande ($\alpha_1$, $\alpha_2$) comprend les sous-étapes suivantes :

   - détermination d'une première valeur de commande approchée ($\tilde{S}_V$) et d'une seconde valeur de commande approchée ($\tilde{\alpha}_1$, $\tilde{\alpha}_2$) en fonction de ladite consigne (S, C, $\alpha$) ;

- évaluation, sur la base dudit modèle, d'au moins une valeur de correction (S', C', $\alpha$') obtenue en appliquant la première valeur de commande approchée ($\tilde{S}_V$) à la lentille (6) et la seconde valeur de commande approchée ($\tilde{\alpha}_1$, $\tilde{\alpha}_2$) à l'ensemble optique (2, 4) ;
- détermination d'une première valeur de commande corrigée (Sv) et d'une seconde valeur de commande corrigée ($\alpha_1$, $\alpha_2$) sur la base d'une comparaison ($\Delta$S, $\Delta$C, $\Delta\alpha$) entre la consigne (S, C, $\alpha$) et la valeur de correction évaluée (S', C', a').

**8.** Procédé de commande selon la revendication 7, comprenant les sous-étapes suivantes :

- évaluation, sur la base dudit modèle, d'au moins une nouvelle valeur de correction obtenue en appliquant la première valeur de commande corrigée à la lentille et la seconde valeur de commande corrigée à l'ensemble optique ;
- détermination d'une nouvelle première valeur de commande corrigée et d'une nouvelle seconde valeur de commande corrigée sur la base d'une comparaison entre la consigne et la nouvelle valeur de correction évaluée.

**9.** Procédé de commande selon la revendication 8, dans lequel les sous-étapes d'évaluation d'au moins une nouvelle valeur de correction et de détermination d'une nouvelle première valeur de commande corrigée et d'une nouvelle seconde valeur de commande corrigée sont réitérées tant que la distance entre la consigne et la nouvelle valeur de correction évaluée est supérieure à un seuil prédéterminé.

**10.** Procédé de commande selon la revendication 6, dans lequel l'étape de détermination d'une première commande ($S_V$) et d'une seconde commande ($\alpha_1$, $\alpha_2$) comprend une sous-étape de lecture de la première commande dans une table de correspondance construite sur la base dudit modèle.

**11.** Procédé de commande selon l'une des revendications 6 à 10, dans lequel l'ensemble optique comprend une seconde lentille (2) et une troisième lentille (4) et dans lequel le modèle tient compte de la distance ($e_1$) séparant la seconde lentille (3) et la troisième lentille (4).

**12.** Dispositif binoculaire d'optométrie comprenant deux dispositifs optiques, dans lequel au moins un des deux dispositifs optiques est un dispositif de compensation visuelle conforme à l'une des revendications 1 à 5.


**Patentansprüche**

**1.** Vorrichtung zur visuellen Kompensation, die es ermöglicht, gemäß einer optischen Beobachtungsachse (X) mit einer optischen Korrektur variabler Leistung zu beobachten, die enthält:

- eine Linse (6), die gemäß der optischen Achse eine abhängig von einer ersten Steuerung (Sv) variable Sphärenleistung hat; und
- eine optische Einheit (2, 4), die gemäß der optischen Achse eine variable Zylinderkorrektur abhängig von mindestens einer zweiten auf die optische Einheit (2, 4) angewendeten Steuerung ($\alpha_1$,$\alpha_2$) erzeugt,
- ein Empfangsmodul (60) mindestens eines Sollwerts (S, C, $\alpha$) für die optische Korrektur;
- ein Bestimmungsmodul (68) der ersten Steuerung (Sv) und der zweiten Steuerung ($\alpha_1$,$\alpha_2$) abhängig vom Sollwert (S, C, $\alpha$),

**dadurch gekennzeichnet, dass** das Bestimmungsmodul konfiguriert ist, die erste und die zweite Steuerung mittels eines Modells zu bestimmen, das den die Linse (6) und die optische Einheit (2, 4) trennenden Abstand ($e_2$) berücksichtigt.

**2.** Vorrichtung zur visuellen Kompensation nach Anspruch 1, wobei das Bestimmungsmodul (68) der ersten Steuerung (Sv) und der zweiten Steuerung ($\alpha_1$,$\alpha_2$) enthält:

- ein Bestimmungsmodul (80) eines ersten angenäherten Steuerwerts ($\tilde{S}_V$) und eines zweiten angenäherten Steuerwerts ($\tilde{\alpha}_1$, $\tilde{\alpha}_2$) abhängig vom Sollwert (S, C, $\alpha$);
- ein Ermittlungsmodul (82), auf der Basis des Modells, mindestens eines Korrekturwerts (S', C', $\alpha$'), der durch Anwendung des ersten angenäherten Steuerwerts ($\tilde{S}_V$) auf die Linse (6) und des zweiten angenäherten Steuerwerts ($\tilde{\alpha}_1$, $\tilde{\alpha}_2$) auf die optische Einheit (2, 4) erhalten wird;
- ein Bestimmungsmodul (84, 86, 88) eines ersten korrigierten Steuerwerts ($\alpha_1$, $\alpha_2$) und eines zweiten korrigierten Steuerwerts (Sv) auf der Basis eines Vergleichs ($\Delta S$, $\Delta C$, $\Delta\alpha$) zwischen dem Sollwert (S, C, $\alpha$) und dem ermittelten Korrekturwert (S', C', a').

3. Vorrichtung zur visuellen Kompensation nach Anspruch 2, wobei das Bestimmungsmodul (68) der ersten Steuerung und der zweiten Steuerung konzipiert ist, den korrigierten ersten Steuerwert ($\alpha_1$,$\alpha_2$) und den korrigierten zweiten Steuerwert (Sv) als erste Steuerung bzw. zweite Steuerung zu verwenden.

4. Vorrichtung zur visuellen Kompensation nach Anspruch 1, wobei das Bestimmungsmodul (68) der ersten Steuerung und der zweiten Steuerung konzipiert ist, die erste Steuerung in einer Entsprechungstabelle abzulesen, die auf der Basis des Modells aufgebaut ist.

5. Vorrichtung zur visuellen Kompensation nach einem der Ansprüche 1 bis 4, wobei die optische Einheit eine zweite Linse (2) und eine dritte Linse (4) enthält, und wobei das Modell den die zweite Linse (2) und die dritte Linse (4) trennenden Abstand ($e_1$) berücksichtigt.

6. Steuerverfahren einer Vorrichtung zur visuellen Kompensation, die es ermöglicht, gemäß einer optischen Beobachtungsachse (X) mit einer optischen Korrektur variabler Leistung zu beobachten, und die eine Linse (6) und eine optische Einheit (2, 4) enthält, das die folgenden Schritte enthält:

- Empfang mindestens eines Sollwerts (S, C, $\alpha$) für die optische Korrektur;
- Bestimmung einer ersten Steuerung (Sv) und einer zweiten Steuerung ($\alpha_1$,$\alpha_2$) abhängig von dem Sollwert (S, C, $\alpha$) mittels eines Modells, das den die Linse (6) und die optische Einheit (2, 4) trennenden Abstand ($e_2$) berücksichtigt;
- Änderung der Sphärenleistung der Linse (6) gemäß der optischen Achse abhängig von der ersten Steuerung (Sv) ; und
- Änderung einer gemäß der optischen Achse von der optischen Einheit (2, 4) erzeugten Korrektur abhängig von der zweiten Steuerung ($\alpha_1$,$\alpha_2$).

7. Steuerverfahren nach Anspruch 6, wobei der Schritt der Bestimmung einer ersten Steuerung (Sv) und einer zweiten Steuerung ($\alpha_1$,$\alpha_2$) die folgenden Teilschritte enthält:

- Bestimmung eines ersten angenäherten Steuerwerts ($\tilde{S}_v$) und eines zweiten angenäherten Steuerwerts ($\tilde{a}_1$,$\tilde{a}_2$) abhängig vom Sollwert (S, C, $\alpha$);
- Ermittlung, auf der Basis des Modells, mindestens eines Korrekturwerts (S', C', $\alpha$'), der durch Anwendung des ersten angenäherten Steuerwerts ($\tilde{S}_v$) auf die Linse (6) und des zweiten angenäherten Steuerwerts ($\tilde{\alpha}_1$,$\tilde{\alpha}_2$) auf die optische Einheit (2, 4) erhalten wird;
- Bestimmung eines ersten korrigierten Steuerwerts (Sv) und eines zweiten korrigierten Steuerwerts ($\alpha_1$,$\alpha_2$) auf der Basis eines Vergleichs ($\Delta S$, $\Delta C$, $\Delta\alpha$) zwischen dem Sollwert (S, C, $\alpha$) und dem ermittelten Korrekturwert (S', C', $\alpha$').

8. Steuerverfahren nach Anspruch 7, das die folgenden Teilschritte enthält:

- Ermittlung, auf der Basis des Modells, mindestens eines neuen Korrekturwerts, der durch Anwendung des korrigierten ersten Steuerwerts auf die Linse und des korrigierten zweiten Steuerwerts auf die optische Einheit erhalten wird;
- Bestimmung eines neuen korrigierten ersten Steuerwerts und eines neuen korrigierten zweiten Steuerwerts auf der Basis eines Vergleichs zwischen dem Sollwert und dem ermittelten neuen Korrekturwert.

9. Steuerverfahren nach Anspruch 8, wobei die Teilschritte der Ermittlung mindestens eines neuen Korrekturwerts und der Bestimmung eines neuen korrigierten ersten Steuerwerts und eines neuen korrigierten zweiten Steuerwerts wiederholt werden, so lange der Abstand zwischen dem Sollwert und dem ermittelten neuen Korrekturwert höher ist als eine vorbestimmte Schwelle.

10. Steuerverfahren nach Anspruch 6, wobei der Schritt der Bestimmung einer ersten Steuerung (Sv) und einer zweiten Steuerung ($\alpha_1$,$\alpha_2$) einen Teilschritt des Ablesens der ersten Steuerung in einer auf der Basis des Modells aufgebauten Entsprechungstabelle enthält.

11. Steuerverfahren nach einem der Ansprüche 6 bis 10, wobei die optische Einheit eine zweite Linse (2) und eine dritte Linse (4) enthält, und wobei das Modell den die zweite Linse (3) und die dritte Linse (4) trennenden Abstand ($e_1$) berücksichtigt.

12. Binokulare Optometrievorrichtung, die zwei optische Vorrichtungen enthält, wobei mindestens eine der zwei optischen Vorrichtungen eine Vorrichtung zur visuellen Kompensation nach einem der Ansprüche 1 bis 5 ist.

**Claims**

1. Visual compensation device allowing observation along an optical axis (X) of observation with an optical correction of variable power, comprising:

   - a lens (6) having, along the optical axis, a variable spherical power that depends on a first setting (Sv); and
   - an optical assembly (2, 4) generating, along the optical axis, a variable cylindrical correction that depends on at least one second setting ($\alpha_1$, $\alpha_2$) applied to said optical assembly (2, 4),
   - a module (60) for receiving at least one setpoint (S, C, $\alpha$) for said optical correction;
   - a determination module (68) for determining the first setting (Sv) and the second setting ($\alpha_1$, $\alpha_2$) depending on said setpoint (S, C, $\alpha$)

   **characterised in that** the determination module is configured to determine the first and the second setting by means of a model taking into account the distance ($e_2$) separating said lens (6) and said optical assembly (2, 4).

2. Visual compensation device according to claim 1, wherein the module (68) for determining the first setting (Sv) and the second setting ($\alpha_1$, $\alpha_2$) comprises:

   - a module (80) for determining an approximate first setting value ($\tilde{S}_V$) and an approximate second setting value ($\tilde{\alpha}_1$, $\tilde{\alpha}_2$) depending on said setpoint (S, C, $\alpha$);
   - a module (82) for evaluating, on the basis of said model, at least one correction value (S', C', $\alpha$') obtained by applying the approximate first setting value ($\tilde{S}_V$) to the lens (6) and the approximate second setting value ($\tilde{\alpha}_1$ ($\alpha_2$) to the optical assembly (2, 4);
   - a module (84, 86, 88) for determining a first corrected setting value ($\alpha_1$, $\alpha_2$) and a second corrected setting value (Sv) on the basis of a comparison ($\Delta$S, $\Delta$C, $\Delta\alpha$) between the setpoint (S, C, $\alpha$) and the evaluated correction value (S', C', a').

3. Visual compensation device according to claim 2, wherein the module (68) for determining the first setting and the second setting is designed to use the first corrected setting value ($\alpha_1$, $\alpha_2$) and the second corrected setting value (Sv) respectively as first setting and second setting.

4. Visual compensation device according to claim 1, wherein the module (68) for determining the first setting and the second setting is designed to read the first setting from a look up table constructed on the basis of said model.

5. Visual compensation device according to one of claims 1 to 4, wherein the optical assembly comprises a second lens (2) and a third lens (4) and wherein the model takes into account the distance ($e_1$) separating the second lens (2) and the third lens (4).

6. Method for controlling a visual compensation device (10) allowing observation along an optical axis (X) of observation with an optical correction of variable power and comprising a lens (6) and an optical assembly (2, 4), comprising the following steps:

   - receiving at least one setpoint (S, C, $\alpha$) for said optical correction;
   - determining a first setting ($S_V$) and a second setting ($\alpha_1$, $\alpha_2$) depending on said setpoint (S, C, $\alpha$) by means of a model taking into account the distance ($e_2$) separating said lens (6) and said optical assembly (2, 4);
   - modifying the spherical power of the lens (6) along the optical axis depending on the first setting ($S_V$); and
   - modifying a cylindrical correction generated along the optical axis by the optical assembly (2, 4) depending on the second setting ($\alpha_1$, $\alpha_2$).

7. Control method according to claim 6, wherein the step of determining a first setting (Sv) and a second setting ($\alpha_1$, $\alpha_2$) comprises the following substeps:

- determining an approximate first setting value ($\tilde{S}_V$) and an approximate second setting value ($\tilde{\alpha}_1$, $\tilde{\alpha}_2$) depending on said setpoint (S, C, $\alpha$);
- evaluating, on the basis of said model, at least one correction value (S', C', $\alpha$') obtained by applying the approximate first setting value ($\tilde{S}_V$) to the lens (6) and the approximate second setting value ($\tilde{\alpha}_1$ $\tilde{\alpha}_2$) to the optical assembly (2, 4);
- determining a corrected first setting value ($S_V$) and a corrected second setting value ($\alpha_1$, $\alpha_2$) on the basis of a comparison ($\Delta$S, $\Delta$C, $\Delta\alpha$) between the setpoint (S, C, $\alpha$) and the evaluated correction value (S', C', a').

8. Control method according to claim 7, comprising the following substeps:

- evaluating, on the basis of said model, at least one new correction value obtained by applying the corrected first setting value to the lens and the corrected second setting value to the optical assembly;
- determining a new corrected first setting value and a new corrected second setting value on the basis of a comparison between the setpoint and the evaluated new correction value.

9. Control method according to claim 8, wherein the substeps of evaluating at least one new correction value and determining a new corrected first setting value and a new corrected second setting value are reiterated as long as the distance between the setpoint and the evaluated new correction value is larger than a preset threshold.

10. Control method according to claim 6, wherein the step of determining a first setting ($S_v$) and a second setting ($\alpha_1$, $\alpha_2$) comprises a sub-step of reading the first setting from a look up table constructed on the basis of said model.

11. Control method according to one of claims 6 to 10, wherein the optical assembly comprises a second lens (2) and a third lens (4) and wherein the model takes into account the distance ($e_1$) separating the second lens (3) and the third lens (4).

12. Optometric binocular device comprising two optical devices, wherein at least one of the two optical devices is a visual compensation device according to one of claims 1 to 5.

# Fig.1

# Fig.2

# Fig.5

**Fig.3**

**Fig.4**

**Fig.6**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 2004032568 A
- WO 2007026368 A
- EP 2034338 A